(19) Europäisches Patentamt
European Patent Office
Office européen des brevets

(11) Veröffentlichungsnummer: **0 140 321**
**B1**

(12) # EUROPÄISCHE PATENTSCHRIFT

(45) Veröffentlichungstag der Patentschrift: **12.04.89**

(51) Int. Cl.⁴: **A 23 K 1/16**, C 07 H 13/00, C 07 H 13/06

(21) Anmeldenummer: **84112708.7**

(22) Anmeldetag: **22.10.84**

(54) **Verwendung N-glycosylierter Carbonsäureamid-Derivate als Wachstumsförderer in der Tierernährung.**

(30) Priorität: **03.11.83 DE 3339694**

(43) Veröffentlichungstag der Anmeldung:
**08.05.85 Patentblatt 85/19**

(45) Bekanntmachung des Hinweises auf die Patenterteilung:
**12.04.89 Patentblatt 89/15**

(84) Benannte Vertragsstaaten:
**BE CH DE FR GB IT LI NL**

(56) Entgegenhaltungen:
**EP-A- 0 091 645**
**DE-A- 2 413 720**
**DE-A- 2 521 800**
**US-A- 4 320 150**

**PATENTS ABSTRACTS OF JAPAN, Band 7, Nr. 22 (C-148)[1167], 28. Januar 1983; & JP-A-57 179 174 (TAKEDA YAKUHIN KOGYO K.K.) 04-11-1982**

(73) Patentinhaber: **BAYER AG, Konzernverwaltung RP Patentabteilung, D-5090 Leverkusen 1 Bayerwerk (DE)**

(72) Erfinder: **Lockhoff, Oswald, Dr., Morgengraben 14, D-5000 Koeln 80 (DE)**
Erfinder: **Stadler, Peter, Dr., Am Ideck 8, D-5657 Haan 1 (DE)**
Erfinder: **Scheer, Martin, Dr., Herberts-Katernberg 7, D-5600 Wuppertal 1 (DE)**
Erfinder: **Berschauer, Friedrich, Dr., Claudiusweg 9, D-5600 Wuppertal 1 (DE)**
Erfinder: **de Jong, Anno, Dr., Stockmannsmuehle 46, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von Verbindungen der allgemeinen Formel I

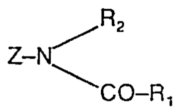

(I)

als Wachstumsförderer in der Tierernährung sowie Tiernahrung, Prämixe und andere Mittel, die Verbindungen der Formel I enthalten.

In Formel I bedeutet $R_1$ Wasserstoff oder einen gegebenenfalls substituierten geradkettigen oder verzweigten, gesättigten oder einfach bzw. mehrfach ungesättigten Alkylrest mit einem bis zu 30 Kohlenstoffatomen, wobei dieser Rest $R_1$ auch durch bis zu 5, vorzugsweise 1 oder 2 O, S und/oder N unterbrochen sein kann, mit der Maßgabe, daß $-COR_1$ keine Acylgruppe mit 1–5 C-Atomen darstellt, wenn $R_2$ Alkyl mit 10–20 C-Atomen bedeutet.

Bei einer Unterbrechung der Kette durch N trägt dieser Stickstoff entweder H oder einen $C_1$–$C_{20}$-, vorzugsweise $C_1$–$C_5$-Alkylrest oder einen –CO-Alkylrest, wobei diese Alkylgruppe 1–20, vorzugsweise 1–5 C-Atome aufweist.

Bevorzugt werden Verbindungen der Formel I, in denen $R_1$ einen Alkyl- oder Alkenylrest mit 1 bis 21 Kohlenstoffatomen vorzugsweise mit 9 bis 21 C-Atomen dar. Beispielhaft für gesättigte Reste seien hier genannt Methyl, Ethyl, Propyl, i-Propyl, Butyl, i-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Docosyl, Ethylpentyl, Methyldecyl, i-Propyldecyl oder Methyltridecosyl bedeutet, für den erfindungsgemäßen Zweck eingesetzt.

Ungesättigte Reste in den erfindungsgemäß verwendeten Erfindungen sind beispielsweise Ethylen, Propenyl-1, Propenyl-2, i-Butenyl, Butenyl-1, Butenyl-2, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Decenyl, 5-Decenyl, 9-Decenyl, 8-Heptadecenyl, 1,3-Butadienyl, 1,3-Pentadienyl, 1,4-Pentadienyl, 2,4-Pentadienyl, 8,11-Heptadecandienyl, 8,11,14-Heptadecantrienyl. Im allgemeinen sind die längerkettigen, ungesättigten Reste bevorzugt, speziell die ein- oder zweifach ungesättigten Alkenyle mit 7–21 C-Atomen.

Die ungesättigten Kohlenwasserstoffreste können dabei als reine cis- oder trans-Isomere oder auch als Isomerengemische vorliegen.

Beispiele für erfindungsgemäß verwendete Verbindungen, in denen die Kohlenwasserstoffreste $R_1$ in Formel I durch O, S und N bzw. entsprechende Atomgruppierungen unterbrochen oder durch diese Atome enthaltende Gruppen oder durch Halogenatome substituiert werden, sind Methoxyethyl-, Hydroxyheptadecenyl-, Oxobutyl-, Aminodecyl-, N-Methyl-aminodecyl-, Fluormethyl-, β-Hydroxytridecyl- oder Mercaptoethylrest.

Die Kohlenwasserstoffreste $R_1$ in Formel I können auch Phenylreste enthalten, wobei diese Phenylreste gegebenenfalls auch durch einen bis drei Substituenten aus der Reihe Nitro, Niedrigalkyl oder durch 1–5 Halogenatome substituiert sein können.

$R_2$ in Formel I steht für Wasserstoff oder einen geradkettigen oder verzweigten, gesättigten oder einfach bzw. mehrfach ungesättigten Alkyl-, Cycloalkyl-, Alkylcycloalkyl- oder einen Aralkylrest mit bis zu 30 Kohlenstoffatomen, wobei im Rest $R_2$ auch einzelne Methylen- oder Methingruppen durch bis zu 5 Sauerstoff- oder Schwefelatome oder N, NH oder N-Niederalkylgruppierungen ersetzt sein können. Einzelne Wasserstoffatome in den Alkyl, Cycloalkyl oder Aralkylresten können auch durch bis zu 5 Sauerstoff enthaltene Gruppen oder Halogenatome substituiert sein.

Beispiele, in welchen $R_2$ in Formel I für einen geradkettigen oder verzweigten, gegebenenfalls einfach oder mehrfach ungesättigten Alkylreste steht die für $R_1$ genannten.

Besonders seien genannt Ethyl, Propyl, Hexyl, Decyl, Dodecyl, Tetradecyl, Hexadecyl, Octadecyl, Docosyl, Myricyl, Ethylhexyl, Isobutyl, Propenyl, Octenyl, Hexadienyl, Docosenyl, Dimethylhexenyl und 2-(Cyclohexyl)-ethyl. Die ungesättigten Kohlenwasserstoffe können als reine cis- oder trans-Isomere oder als Isomerengemisch vorliegen. Durch Sauerstoffatome enthaltende Gruppen substituierte Kohlenwasserstoffreste $R_2$ sind beispielsweise Hydroxypropyl und Hydroxydimethyloctyl, durch Sauerstoffatome unterbrochene Kohlenwasserstoffreste sind beispielsweise Alkoxyalkylreste wie Metoxybutyl oder Butoxypropyl, ein durch N und O unterbrochener Rest ist beispielsweise 2-(N-morpholino)-ethyl und als Beispiel für einen halogensubstituierten Kohlenwasserstoffrest sei Trifluormethylethyl genannt.

Aralkyl für $R_2$ in Formel I ist zum Beispiel Arylniedrigalkyl wie Benzyl, Phenethyl oder Phenylhexyl, wobei der Phenylkern gegebenenfalls einfach oder mehrfach, vorzugsweise ein- oder zweifach, zum Beispiel durch Niederalkyl, Trifluormethyl, Halogen, Hydroxy oder Niedrigalkoxy substituiert sein kann.

Unter Niedrigalkyl oder -alkoxy werden solche Reste verstanden, die 1–5, vorzugsweise 1–3 C-Atome enthalten.

Z in den erfindungsgemäß verwendeten Verbindungen der Formel I bedeutet einen Glykosylrest, der bei den erfindungsgemäßen Verbindungen immer über das anomere Kohlenstoffatom an den Amidstickstoff gebunden ist, wobei unter Glykosylreste erfindungsgemäß besonders verstanden werden Mono-, Di- und Oligosaccharidreste, besonders Mono- und Disaccharide, in denen gegebenenfalls eine oder mehrere Hydroxylgruppen durch Aminogruppen, Acylamidogruppen, Azidogruppen, Wasserstoff, Nitro, Thiolgruppen oder Niedrigalkoxy oder Halogen ersetzt sein können und die Glykosylreste auch in Form der entsprechenden Ulosen, Ulosederivate, Uronsäuren oder von Uronsäurederivaten vorliegen können.

Die Glykosylreste Z in Formel I liegen erfindungsgemäß bevorzugt in der Pyranosyl- oder der Furanosylform vor, wobei die betreffenden Monosaccharid-, Disaccharid oder Oligosaccharidreste bevorzugt aus Pentosen, Hexosen und Heptosen aufgebaut sind.

Beispiele für Monosaccharidreste in den erfindungsgemäß verwendeten Verbindungen sind Glucopyranosyl-, Galactopyranosyl-, Mannopyranosyl-, Glucofuranosyl-, Ribofuranosyl, Arabinopyranosyl- oder auch Lyxopyranosyl- oder auch D-Glycero-D-gluco-heptopyranosylreste. Als Beispiel für Di- und Oligosaccharidreste können genannt werden Maltosyl-, Maltrotriosyl-, Maltotetraosyl-, Lactosyl-, Cellobiosyl-, Melibiosyl- oder 6-O-(α- oder β-Ribofuranosyl)-Glucopyranosylreste, d. h. also Kohlenhydratsysteme, die aus Zukkern unterschiedlicher C-Zahl aufgebaut sind und bei denen die Zucker in der Pyranose und/oder Furanoseform vorliegen können. Die glykosidischen Bindungen zwischen den einzelnen Zukkerbausteinen können in der α- und/oder β-Form vorliegen und die glykosidische Verknüpfung der einzelnen Zuckerbausteine kann von einem anomeren Kohlenstoffatom ausgehend sowohl über die primäre OH-Gruppe als auch über eine der sekundären Hydroxylgruppen des als Aglykon fungierenden Saccharidteils erfolgen.

Als Beispiele für Mono-, Di- und Oligosaccharidreste, in denen eine oder mehrere OH-Gruppen durch Aminogruppen, Acylamidogruppen, Azidogruppen, Wasserstoff, Nitro, Thiolgruppen, Niedrigalkoxy oder Halogen ersetzt sind, seien für die erfindungsgemäß verwendeten Verbindungen genannt

2-Acetylamino-2-desoxyglucopyranosyl-,
2-Amino-2-desoxyglucopyranosyl-,
4-Azido-4-desoxy-glucopyranosyl-,
4-Stearoylamido-4-desoxy-D-glucopyranosyl-,
4-Dodecoylamido-4-desoxy-D-glucopyranosyl-,
6-Hexadecanoylamido-6-desoxy-D-galactopyranosyl-,
2,6-Diamino-2,6-didesoxyglucopyranosyl-,
6,6′-Diamino-6,6′-didesoxymaltosyl-,
6-Amino-6,6′-didesoxylactosyl,
6-Desoxymannopyranosyl-,
2-Desoxyribofuranosyl-, Fucosyl,
5-Fluor-5-desoxyribofuranosyl-,
6-O-Methylglucopyranosyl,
6-Desoxy-6-thioglucopyranosyl,
3-Desoxy-3-nitrogalactopyranosyl.

Liegen die Glykosylreste in Form von Uronsäuren oder Uronsäurederivaten vor, so handelt es sich um Glykuronsäuren mit freier Carboxylgruppe oder mit durch Alkyl veresteter Carboxylgruppe oder um Glykuronamidderivate mit unsubstituiertem oder substituiertem Stickstoffatom. Beispiele für entsprechende Zucker sind Galacturonsäure, Glucuronsäuremethylester oder auch N-Dodecylglucuronamid.

Die Verbindungen der Formel I enthalten mehrere chirale C-Atome und liegen als optische reine Diasteromere oder als Diasteromerengemische vor. Die erfindungsgemäß verwendeten Verbindungen der Formel I sind also Carbonsäureamide oder N-alkylierte bzw. N-aralkylierte Carbonsäureamide, die jeweils am Amidstickstoff N-glykosidisch – d. h. also über das anomere Kohlenstoffatom gebunden – einen einfachen oder modifizierten Mono-, Di- oder Oligosaccacharidrest tragen.

Ganz besonders bevorzugte erfindungsgemäß verwendete Verbindungen sind die durch Ausführungsbeispiele belegten, insbesondere die der Beispiele 12, 13, 14, 15, 16, 17, 18, 23, 24, 30, 31, 37, 42, 43, 44, 45, 46, 48, 49, 50, 53, 54, 55, 56, 57, 58, 59 und 60.

Zur Herstellung der erfindungsgemäß verwendeten Verbindungen setzt man die von Z in Formel 1 umfaßten Zucker entweder in freier, d. h. ungeschützter Form, oder in Form geschützter, gegebenenfalls aktivierter Derivate zunächst mit einer Aminoverbindung $R_2$–$NH_2$, entweder in freier Form oder in Form eines geeigneten Säureadditionssalzes mit der vorstehend beschriebenen Bedeutung für $R_2$, um und acyliert anschließend das dabei erhaltene Glykosylamin mit einem – wie bei Acylierungsreaktionen üblich – aktivierten, an funktionellen Gruppen gegebenenfalls geschützten, Carbonsäurederivat, spaltet in dem so erhaltenen Reaktionsprodukt gegebenenfalls vorhandene Schutzgruppen ab und erhält auf diese Weise die Verbindungen der Formel I, die falls erforderlich, durch Chromatographie, Umkristallisieren, Extraktion o. ä. gereinigt werden können.

In einer bevorzugten Ausführungsform wird in an sich bekannter Weise in einem ersten Verfahrensschritt der unblockierte Zucker Z–OH, wobei OH für die anomere Hydroxylgruppe steht, mit der in Formel I beschriebenen Bedeutung von Z in einem geeigneten Lösungsmittel, oder auch ohne Lösungsmittel, gegebenenfalls in Gegenwart eines Katalysators bei Temperaturen zwischen 0 °C und 80 °C mit 1 bis 10 Äquivalenten des betreffenden Amins $R_2$–$NH_2$ umgesetzt und man erhält gewöhnlich in hohen Ausbeuten nach Aufarbeitung die betreffenden Glykosylamine Z–NH–$R_2$ als amorphe oder kristalline Feststoffe oder als zähe Sirupe.

Im zweiten Verfahrensschritt wird dann das Glykosylamin Z–NH–$R_2$ mit 1–10 Äquivalenten eines Carbonsäurederivates der Formel $R_1$–CO–X, in der $R_1$ die obengenannte Bedeutung besitzt und X Halogen oder eine bei Acylierungsreaktionen gebräuchliche Abgangsgruppe, vorzugsweise einen aktivierenden Esterrest, oder eine Gruppe O–OC–$R_1$ mit der obigen Bedeutung für $R_1$ bezeichnet, umgesetzt, wobei man in einem organischen oder wäßrig-organischen Lösungsmittel bei Temperaturen zwischen 0 °C und 50 °C, gegebenenfalls in Gegenwart einer Base, arbeitet und nach beendeter Umsetzung das Reaktionsprodukt in üblicher Weise aufarbeitet.

Für den Fall, daß im Kohlenhydratrest Z eine oder mehrere freie Aminogruppen vorhanden sind, werden diese vor der Umsetzung mit dem Amin $R_2$–$NH_2$, in an sich bekannter Weise mit einer Aminoschutzgruppe versehen.

Als Aminoschutzgruppen kommen solche in der Zucker- und Peptidchemie üblicherweise verwendeten Gruppen in Frage (siehe zum Beispiel HOUBEN-WEYL, Methoden der organischen Chemie, Band XV, Georg Thieme Verlag, Stuttgart, 1974), die einerseits unter den gegebenen Reaktionsbedingungen stabil sind, die andererseits aber nach der Herstellung des betreffenden N-Glykosids und dessen anschließender Acylierung mit einem Carbonsäurederivat $R_1$–CO–X, bei der vorstehend angegebenen Bedeutung für $R_1$, so selektiv wieder abgespalten werden können, daß das gewünschte Endprodukt der Formel I erhalten wird, d. h. ohne daß die im Endprodukt der Formel I enthaltende Acylaminogruppe nach gespalten wird. Bevorzugte Beispiele sind Acylgruppe vom Typ

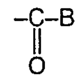

wobei B Trichlormethyl oder Trifluormethyl bedeutet oder vom Typ

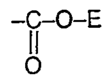

wobei E z. B. für Trichlorethyl oder tertiär-Butyl steht oder auch Sulfenylgruppen vom Typ

–S–G

wobei G für Phenyl, substituiertes Phenyl oder Di- oder Triphenylmethyl steht und ‹substituiertes Phenyl› einen Phenylrest bezeichnet, der durch einen bis drei Substituenten aus der Reihe Nitro, Niedrigalkyl oder der durch 1 bis 5 Halogenatome, vorzugsweise Chloratome, substituiert ist. Als Beispiele seien der 2,4,5-Trichlorphenylsulfenyl und der o-Nitrophenylsulfenylrest genannt.

Die Einführung dieser Schutzgruppen in Aminoverbindungen und ihre spätere Abspaltung zur Freisetzung der gewünschten Aminogruppen sind bekannt und beispielsweise in der oben zitierten Literaturstelle beschrieben.

In einer anderen Ausführungsform des Verfahrens zur Herstellung von solchen Produkten der Formel I in denen im Glykosylrest Z eine oder mehrere freie Aminogruppen vorhanden sind, setzt man Zuckerderivate Z–OH als Ausgangsprodukte ein, in welchen die Aminogruppe oder die Aminogruppen zunächst in Form von Azidoresten, d. h. also in verkappter Form vorliegen. Im abschließenden Schritt der Herstellung der Verbindungen der Formel I werden diese Azidogruppen reduktiv in an sich bekannter Weise in Aminogruppen umgewandelt, wobei darauf geachtet wird, daß solche Reduktionsmittel verwendet werden, die andere gegebenenfalls im Molekül vorhandene reduktionsempfindliche Gruppen nicht angreifen.

Entsprechende Azidozucker und deren Herstellung sind bekannt (siehe zum Beispiel Methods in Carbohydrate Chemistry, Vol. I, 242–246 Academic Press, 1962 New York a. London). Zur Reduktion können verwendet werden, Hydriddonatoren wie z. B. Natriumboranat oder Lithiumalanat, katalytisch erregter Wasserstoff oder auch Triphenylphosphin in Methanol/Amoniak/Pyridin oder auch Schwefelwasserstoff oder Mercaptane in protischen Solventien.

Als Lösungsmittel können alle gängigen organischen Solventien, vorzugsweise niedrige Alkanole, oder aber auch Wasser oder wäßrige Alkanole verwendet werden.

Die Reaktionen werden gegebenenfalls unter Zusatz von organischen Säuren wie Essigsäure oder anorganischen Säuren wie Schwefelsäure oder unter Zusatz organischer Basen wie zum Beispiel Pyridin oder anorganischer Basen wie zum Beispiel Ammoniak durchgeführt. Man arbeitet bei Temperaturen zwischen 0 und 120 °C vorzugsweise 10 °C bis 40 °C, gegebenenfalls unter erhöhtem Druck und/oder Intergas.

Für den Fall, daß in den Verbindungen der Formel I im Kohlenhydratteil Z eine oder mehrere OH-Gruppen durch eine oder mehrere Acylamidogruppen ersetzt sind, werden die Zucker Z–OH von vornherein in Form der entsprechenden Acylamidozucker eingesetzt. Die Acylamidozucker werden dann am anomeren Zentrum mit den obigen angegebenen Aminen zunächst zu den entsprechenden Acylamidoglycosylaminen umgesetzt und im zweiten Reaktionsschritt an der C-1 Aminogruppe des Zuckerteils acyliert zu N-(Acylamidoglycosyl)amiden der Formel I.

Eine andere Verfahrensweise zur Darstellung von Verbindungen der Formel I, in der Z für einen mit einer oder mehreren Acylamidogruppen substituierten Zuckerrest steht, besteht darin, daß man Amino-desoxyzucker, die die Aminogruppe an einem anderen als dem anomeren Kohlenstoffatom tragen, mit Aminen der Formel $R_2$–$NH_2$ umsetzt zu (Amino-desoxy-glycosyl)-aminen, welche dann im zweiten Reaktionsschritt zweifach oder gegebenenfalls mehrfach acyliert werden zu N-(Acylamidoglycosyl)-amiden der Formel I

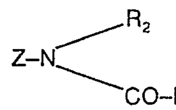

Ferner ist es auch möglich, die Verbindungen der Formel I, in der Z für einen mit einer oder mehreren Acylamidogruppen substituierten Zuckerrest steht, zu erhalten, indem man in Derivaten der Formel I, in der Z zunächst einen durch eine oder mehrere temporäre Amino-Schutzgruppen vom vorstehend beschriebenen Typ

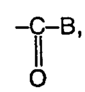, 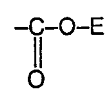 oder –S–G

blockierten Aminozucker darstellt, die temporäre Amino-Schutzgruppen nach den üblichen Methoden abspaltet zu den entsprechenden N-(Aminodesoxyglycosyl)-amiden und diese dann umsetzt mit aktivierten Carbonsäurederivaten zu den entsprechenden N-(Acylamidoglycosyl)-amiden der Formel I

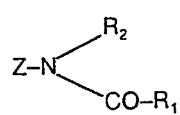

Eine andere Verfahrensweise zur Darstellung von N-(Acylamidoglycosyl)-amiden der Formel I besteht darin, daß man N-(Azidoglycosyl)-amide der Formel I nach den üblichen Methoden umsetzt zu den N-(Aminoglycosyl)-amiden der Formel I

und diese dann mit aktivierten Carbonsäurederivaten acyliert zu N-(Acylamidoglycosyl)-amiden der Formel I.

Der erste Verfahrensschritt bei der Herstellung der Verbindungen der Formel I ist somit die Umsetzung eines Zuckers Z–OH, mit einem Amin des Typs $R_2$–$NH_2$ am anomeren Kohlenstoffatom unter Wasserabspaltung zu dem betreffenden Glykosylamin.

Amine $R_2$–$NH_2$, die bei Raumtemperatur flüssig sind, können mit dem Zucker direkt, d. h. ohne Lösungsmittel umgesetzt werden. Hierbei arbeitet man bei Temperaturen zwischen 0 °C und 100 °C vorzugsweise bei 25 °C bis 70 °C. Als Katalysatoren sind Mineralsäuren wie zum Beispiel Salzsäure, Schwefelsäure oder Salpetersäure oder kurzkettige Carbonsäuren wie Essigsäure oder Propionsäure geeignet, die man in Mengen von 0,001 bis 0,05 Äquivalenten einsetzt.

In jedem Fall ist es möglich, und bei Aminen $R_2$–$NH_2$, die bei Raumtemperatur fest sind auch zu bevorzugen, die Herstellung der Glykosylamine in Gegenwart eines Lösungsmittels durchzuführen. Man arbeitet dann vorzugsweise in Gegenwart eines unter den Reaktionsbedingungen inerten Verdünnungsmittels, welches vorzugsweise so beschaffen ist, daß sich zumindest entweder die Reaktionspartner oder das Reaktionsprodukt in ihm lösen.

In Frage kommen Alkohole wie Methanol, Ethanol, Propanol-1 und Propanol-2, Ether wie Tetrahydrofuran und Dioxan, sowie auch Dimethylformamid, wobei – außer bei der Verwendung der Alkohole – der Zusatz von Wasser zu bevorzugen ist. Darüber hinaus ist vorzugsweise bei kurzkettigen Aminen $R_2$–$NH_2$ auch Wasser allein als Lösungsmittel geeignet. Es kann auch von Vorteil sein, die Alkanole im Gemisch mit Wasser zu verwenden.

Die Reaktionstemperaturen bei Verwendung von Lösungsmitteln bei der Herstellung der Glykosylamine liegen zwischen –10 °C und 120 °C vorzugsweise zwischen 30 °C und 70 °C.

Das betreffende Verdünnungsmittel kann wahlweise vor oder während der Reaktion zugegeben werden. Bei langkettigen Aminen $R_2$–$NH_2$ ist eine Zugabe vor der Reaktion zu bevorzugen.

Die wie vorstehend beschrieben herstellten Glykosylamine kristallisieren entweder direkt oder nach Abkühlen aus und können durch Zusatz geeigneter, vorzugsweise weniger polarer Hilfslösungsmittel wie Aceton, Diethylether, Cyclohexan, Essigester oder Petrolether, gegebenenfalls unter Kühlung, ausgefällt oder zur Kristallisation gebracht werden, gegebenenfalls vorhandenes überschüssiges Amin $R_2$–$NH_2$ kann durch Waschen oder Umkristallisieren des Produktes auf an sich bekannter Weise entfernt werden.

Der zweite Verfahrensschritt bei der Herstellung der Verbindungen der Formel I ist die selektive N-Acylierung eines wie vorstehend beschrieben erhaltenen Glykolsylamins mit einem Carbonsäurederivat der Formel $R_1$–CO–X mit der vorstehend angegebenen Bedeutung von $R_1$ und X. Als an sich bekannte Carbonsäurederivate R–CO–X sind zu bevorzugen Anhydride, aktivierte Ester und Säurehalogenide, vorzugsweise Chloride.

Diese Acylierungsmittel werden vorzugsweise in Gegenwart eines Verdünnungsmittels mit den Glykosylaminen umgesetzt, in welchem die Reaktionspartner vollständig oder auch nur teilweise gelöst sind.

Es kommen organische oder anorganische Solventien in Frage, vorzugsweise solche, die unter den Reaktionsbedingungen Nebenreaktionen möglichst herabsetzen oder verhindern. Man kann sowohl in organischen Lösungsmitteln wie Ethern, z. B. Tetrahydrofuran und Dioxan, oder Alkoholen z. B. Ethanol und Propanol, oder Ketonen, z. B. Aceton oder Methylethylketon, oder in Dimethylformamid, Essigester oder Pyridin als auch in Mischungen dieser Lösungsmittel untereinander und/oder mit Wasser arbeiten. Die Verwendung von wasserfreien Solventien ist im allgemeinen zu bevorzugen.

Die Acylierungsmittel $R_1$–CO–X werden in 1–10 Äquivalenten, bezogen auf Glykosylamin eingesetzt, wobei die Verwendung von 1–3 Äquivalenten zu bevorzugen ist.

Die Acylierungsreaktionen können, vorzugsweise bei Verwendung von Säurehalogeniden und -anhydriden, in Gegenwart basischer Hilfsstoffe durchgeführt werden. Verwendet werden können alle in der organischen Synthese üblichen basischen Verbindungen wie z. B. tertiäre aliphatische oder auch aromatische Amine oder aber Alkali- und Erdalkalihydroxide bzw. -carbonate wie Natronlauge, Natriumcarbonat oder Calciumcarbonat.

Die Acylierungen werden bei Temperaturen zwischen etwa –30 °C und +80 °C, vorzugsweise zwischen –10 °C und +20 °C, durchgeführt.

Die auf diese Weise erhaltenen Amide werden nach an sich bekannten Verfahren in Form von kristallinen oder amorphen Feststoffen oder als zähe Sirupe isoliert und, wenn notwendig, durch Umkristallisation, Chromatographie, Extraktion usw. gereinigt.

Im Falle von Verbindungen mit geschützten Aminogruppen im Glykosylteil werden die Schutzgruppen in an sich bekannter Weise abgespalten.

Das folgende Formelschema soll eine der bevorzugten Ausführungsformen der Darstellung von Verbindungen der Formel I beispielhaft erläutern:

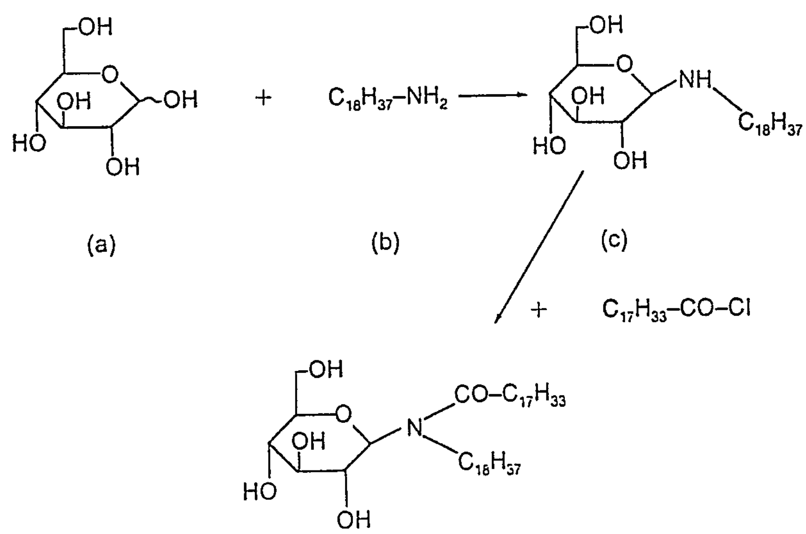


I

Im ersten Verfahrensschritt wird Glucose (a) mit Octadecylamin (b) zu N-Octadecyl-β-D-Glucopyranosylamin (c) umgesetzt, das im zweiten Verfahrensschritt mit Ölsäurechlorid zu N-Octadecyl-N-oleyl-β-D-glucopyranosylamin (I) acyliert wird.

Erfindungsgemäß können auch Salze der Verbindungen der Formel I verwendet werden. Dabei handelt es sich in erster Linie um üblicherweise physiologisch unbedenkliche, nicht toxische Salze, z. B. Alkalimetall- oder Ammoniumsalze.

N-glycosylierte Carbonsäureamid-Derivate und ihre Verwendung zur Beeinflussung der körpereigenen Abwehr sind bereits bekannt geworden (EP-A-91 645). Es ist jedoch nichts darüber bekannt, daß sich diese Verbindungen auch zur Förderung und Beschleunigung des Wachstums von Tieren einsetzen lassen.

Die Glycosylamide der Formel (I) weisen überraschenderweise die Eigenschaft auf, bei Tieren das Wachstum zu fördern und zu beschleunigen, so daß diese Verbindungen in allen Bereichen der Tierzucht und Tierhaltung zu den genannten Zwecken eingesetzt werden können.

Die Wirksamkeit der erfindungsgemäß verwendeten Verbindungen ist hierbei weitgehend unabhängig von der Art und dem Geschlecht der Tiere. Besonders wertvoll erweisen sich die erfindungsgemäßen Verbindungen der Formel (I) bei der Aufzucht und Haltung von Jung- und Masttieren. Als Tiere, bei denen die Verbindung zur Förderung und Beschleunigung des Wachstums eingesetzt werden kann, seien beispielsweise folgende Nutz- und Ziertiere genannt:

Warmblüter wie Rinder, Schweine, Pferde, Schafe, Ziegen, Katzen, Hunde, Kaninchen, Pelztiere, z. B. Nerze und Chinchilla, Geflügel, z. B. Hühner, Gänse, Enten, Truthähne, Broiler, Tauben, Papageien und Kanarienvögel und Kaltblüter, wie Fische, z. B. Karpfen und Reptilien, z. B. Schlangen.

Bevorzugte Anwendung finden Glycosylamide der Formel (I) bei der Aufzucht und Haltung von Wiederkäuern wie Kälber, Ziegen, Schafe sowie bei Schweinen und Küken.

Die Menge von Glycosylamiden der Formel (I), die den Tieren zur Erreichung des gewünschten Effektes verabreicht wird, kann weitgehend variiert werden. Sie liegt vorzugsweise bei etwa 0,1 bis 500, insbesondere 0,1 bis 100 mg/kg Körpergewicht pro Tag. Die Dauer der Verabreichung kann von wenigen Stunden oder Tagen bis zu mehreren Jahren betragen. Die passende Menge Wirkstoff sowie die passende Dauer der Verabreichung hängen insbesondere von der Art, dem Alter, dem Geschlecht, dem Gesundheitszustand und der Art der Haltung der Tiere ab und sind durch jeden Fachmann leicht zu ermitteln.

Die Verbindungen werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen. Aus Zweckmäßigkeitsgründen ist in den meisten Fällen eine orale Verabreichung, insbesondere im Rhythmus der Nahrungs- und/oder Getränkeaufnahme der Tiere, vorzuziehen.

Die Verbindungen können als reine Stoffe oder in formulierter Form, also in Mischung mit nichttoxischen inerten Trägerstoffen, als solche sind feste, halbfeste oder flüssige Verdünnungsmittel, Füllstoffe und Formulierungshilfsmittel jeder Art zu verstehen, verabreicht werden.

Glycosylamide der Formel (I) können gegebenenfalls in formulierter Form auch zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwasser zugegeben werden.

Die Verbindungen können nach üblichen Methoden durch einfaches Mischen als reine Stoffe, vorzugsweise in fein verteilter Form oder in formulierter Form in Mischung mit eßbaren nichttoxischen Trägerstoffen, gegebenenfalls auch in Form eines Prämix oder eines Futterkonzentrates, dem Futter und/oder Trinkwasser beigefügt werden.

Das Futter und/oder Trinkwasser kann beispielsweise den erfindungsgemäßen Wirkstoff in einer Konzentration von etwa 0,1 bis 100, insbesondere 0,5 bis 10,0 ppm enthalten. Die optimale Höhe der Konzentration des Wirkstoffes in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineralstoffen enthalten. Das Futter kann sich beispielsweise zusammensetzen aus pflanzlichen Stoffen, z. B. Heu, Rüben, Getreide, Getreidenebenprodukten, tierischen Stoffen, z. B. Fleisch, Fetten, Knochenmehl, Fischprodukten, Vitaminen, z. B. Vitamin A, D-Komplex und B-Komplex, Proteinen, Aminosäuren, z. B. D-Methionin und anorganischen Stoffen, z. B. Kalk und Kochsalz.

Futterkonzentrate enthalten Glycosylamide der Formel (I) neben eßbaren Stoffen, z. B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentrationen können die Wirkstoffe gegebenenfalls auch durch seine Oberfläche bedeckende geeignete Mittel, z. B. mit nichttoxischen Wachsen oder Gelatine vor Luft, Licht und/oder Feuchtigkeit geschützt werden.

Beispiel für die Zusammensetzung eines Kükenaufzuchtfutters, das Glycosylamide der Formel (I) enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

Die Vitamin-Mineral-Mischung besteht aus:

6000 I. E. Vitamin A, 1000 I. E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 mg Mn $SO_4 \times H_2O$, 140 mg Zn $SO_4 \times 7\ H_2O$, 100 mg Fe $SO_4 \times 7\ H_2O$ und 20 mg Cu $SO_4 \times 5\ H_2O$.

Der Wirkstoff-Praemix enthält Glycosylamide der Formel (I) in der gewünschten Menge, z. B. 100 mg und zusätzlich 1 g DL-Methionin sowie so viel Sojabohnenmehl, daß 2,5 g Praemix entstehen.

Beispiel für die Zusammensetzung eines Schweineaufzuchtfutters, das den erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Eierhefe, 50 g Vitamin-Mineral-Mischung für Schweine (Zusammensetzung z. B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z. B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

14-tägige Fütterungsversuche an Küken und 6-wöchige Fütterungsversuche an Broilern, die 0,1 ppm bis 2,0 ppm Verbindung der Formel (I) mit dem Futter enthielten, zeigten deutliche Gewichtszunahmen bei den mit Glycosylamiden der Formel (I) behandelten Tiere im Vergleich zu den ohne Zusatz von Glycosylamiden der Formel (I) ernährten Tieren.

Beispiele

Die Dünnschichtchromatographie (DC) erfolgte auf Kieselgel-DC-Fertigplatten (E. Merck, Darmstadt) und die präparativen Trennungen mit Kieselgel 60 (Merck, Darmstadt).

Laufmittelsysteme: System G $CH_2Cl_2/CH_3OH/$ 15%iges Ammoniumhydroxid im Verhältnis 1/1/1, davon die Unterphase; System E $CH_2Cl_2/CH_3OH$ 20%iges Ammoniumhydroxid im Verhältnis 8/4/1; jeweils Volumenteile.

Beispiel 1:

N-D-Glucopyranosyl-Ölsäureamid

3 g 2,3,4,6-Tetra-O-acetyl-3,D-glucopyranosylamin wurden in 25 ml Tetrahydrofuran gelöst und nach Zugabe von 3,45 g Natriumcarbonat unter starkem Rühren und Kühlen bei 0 °C tropfenweise mit 2,24 g Ölsäurechlorid gelöst und in 5 ml Tetrahydrofuran (THF) versetzt. Nach vollständiger Reaktion (Kontrolle durch Dünnschichtchromatographie = DC im System Toluol : Aceton = 4 : 1) wurde vom Niederschlag abfiltriert, das Filtrat im Vakuum eingedampft und getrocknet. Zur O-Deacetylierung löste man das so erhaltene Rohprodukt in 200 ml einer Lösung aus Methanol/Triethylamin/Wasser = 4 : 3 : 1 (Volumenteile) und beließ für 15 Stunden bei Raumtemperatur. Dann wurde im Vakuum eingedampft und der Rückstand an Kieselgel chromatographiert. Das auf diese Weise erhaltene Titelprodukt hatte einen Rf-Wert von 0.46.

Beispiel 2:

N-Benzyl-β-D-glucopyranosylamin

50 g D-Glucose werden in 1000 ml heißem Ethanol gelöst und nach Zugabe von 89 g Benzylamin

für 48 Stunden bei Raumtemperatur belassen. Dann wird mit Eis gekühlt und das Produkt mit Petrolether gefällt. Es wird abgesaugt, mit Ether gewaschen und im Vakuum getrocknet.
$^1$H-NMR in $CD_3OD$: δ = 7,33 breites Singulett Phenyl-H.

Beispiel 3:
N-Benzyl-N-glucopyranosyl-acetamid

1 g der Verbindung aus Beispiel 2 wird in 10 ml abs. Pyridin bei 0 °C mit 6 ml Acetanhydrid bei Raumtemperatur acetyliert. Man arbeitet wie üblich auf und erhält 1 g N-Acetyl-tetra-O-acetylderivat.
$^1$H-NMR in CD $Cl_3$: δ = 1,9–2,1 m 5 × $\underline{CH_3}$–CO–.

Zur O-Deacetylierung werden 500 ml des Pentaacetats in abs. Methanol mit 10% Natriummethylat deacetyliert und wie üblich aufgearbeitet. Das Produkt fällt als amorpher Feststoff an.
$^1$H-NMR in $CD_3OD$: δ = 7,1–7,4 Phenyl-H.

Beispiel 4:
N-Dodecyl-β-D-glucopyranosylamin

18 g Glucose werden in 50 ml Ethanol bei 70 °C gerührt, dann fügt man 18,5 g Dodecylamin zu, heizt noch bis zur klaren Lösung, läßt auf Raumtemperatur abkühlen und saugt nach 20 Stunden von den ausgefallenen Kristallen ab. Man wäscht mit Ethanol und Ether und trocknet im Vakuum.
Ausbeute = 24 g.
Elementaranalyse: ($C_{18}H_{37}NO_5$ = 347):
ber. C = 62,2%, H = 10,6%, N = 4,0%
gef. C = 62,2%, H = 10,6%, N = 4,2%

Beispiel 5:
N-Dodecyl-N-β-D-glucopyranosyl-acetamid

Die Herstellung erfolgt analog Beispiel 3.
Elementaranalyse:
ber.: C = 61,7%, H = 10,0%, N = 3,6%
gef.: C = 60,8%, H = 9,9%, N = 3,8%

Beispiel 6:
N-Glucopyranosyl-N-propyl-ölsäureamid

11 g N-Propyl-D-glucopyranosylamin werden in 90 ml Tetrahydrofuran (THF) mit 21 g Soda gerührt, dann tropft man 1 Äquivalent Ölsäurechlorid in 20 ml THF langsam unter Kühlen dazu. Nach vollständiger N-Acylierung (Kontrolle durch DC im Laufmittelsystem $CH_2Cl_2/CH_3OH$ = 13 : 1) wird vom Niederschlag abgesaugt, mit THF nachgewaschen, die Filtrate im Vakuum eingedampft und der erhaltene Sirup an Kieselgel zur Nachreinigung chromatographiert. Entwicklung der Säule mit $CH_2Cl_2/CH_3OH$ = 15 : 1.

Die Fraktionen, die die Titelverbindung rein enthalten, werden vereinigt. Das Lösungsmittel wird im Vakuum entfernt. Ausbeute: 3,3 g.
Rf.-Wert: 0,34 in $CH_2Cl_2/CH_3OH$ = 15 : 1
$[\alpha]_D^{20}$ = + 7,5° (c=1,0 $CH_2Cl_2$)

Beispiel 7:
N-Glucopyranosyl-N-hexyl-ölsäureamid

Die Herstellung erfolgt ausgehend von N-Hexyl-D-glucopyranosylamin wie im Beispiel 6 beschrieben. Säulenchromatographie mit $CH_2Cl_2/CH_3OH$ = 13 : 1.
Ausbeute: 9,2 g Reinprodukt.
Rf.-Wert = 0,38 in $CH_2Cl_2/CH_3OH$ = 13 : 1
$[\alpha]_D^{20}$ = + 5,8 °C (c = 0,94 in $CH_2Cl_2$)

Beispiel 8:
N-Glucopyranosyl-N-(n-3,3,3-trifluor-propyl)-ölsäureamid

3,6 g Glucose und 0,8 ml 0,5 N Salzsäure und 4,6 g n-3,3,3-Trifluorpropylamin werden für 25 Minuten auf 75 °C unter Rühren erhitzt. Nach Abkühlen kristallisiert das N-Glucosid aus, wird mit Ether gewaschen und im Vakuum getrocknet.
Ausbeute: 4,1 g.

Die N-Acylierung mit Ölsäurechlorid erfolgt analog Beispiel 6. Säulenchromatographie mit $CH_2Cl_2/CH_3OH$ = 15 : 1.
Ausbeute: 2,7 g.
$[\alpha]_D^{20}$ = + 7,6° (c = 1,0 in $CH_2Cl_2$)

Beispiel 9:
N-(2-Ethyl-hexyl)-N-glucopyranosyl-ölsäureamid

Die Umsetzung von Glucose mit 2-Ethyl-hexylamin erfolgt analog Beispiel 8. Die N-Acylierung mit Ölsäurechlorid wird analog Beispiel 6 durchgeführt. Säulenchromatographie mit $CH_2Cl_2/CH_3OH$ = 15 : 1.
Rf.-Wert der Titelverbindung: 0,44 in $CH_2Cl_2/CH_3OH$ = 15/1.

Beispiel 10:
N-(3-Butoxy-propyl)-N-glucopyranosyl-ölsäureamid

Herstellung des N-Glycosids und N-Acylierung wie bei Beispiel 8 bzw. Beispiel 6 beschrieben.
Rf.-Wert: 0,29 Laufmittelsystem $CH_2Cl_2/CH_3OH$ = 10/1.

Beispiel 11:
N-Dodecyl-N-glucopyranosyl-stearinsäureamid

100 g N-Dodecyl-β-D-glucopyranosylamin aus Beispiel 4 werden in 765 ml THF gelöst und in Gegenwart von 32 g Triethylamin unter Kühlen tropfenweise mit 80 g Stearinsäurechlorid versetzt.

Zur Aufarbeitung wird filtriert und das Lösungsmittel in Vakuum entfernt.

Ebenso wird hergestellt N-Dodecyl-N-glucopyranosyl-Ölsäureamid.

Beispiel 12:
N-Decyl-N-glucopyranosyl-Ölsäureamid

18 g D-Glucose und 50 ml Ethanol werden bei 70 °C mit 15,7 g Decylamin bis zur klaren Lösung gerührt. Dann läßt man auf Raumtemperatur abkühlen, saugt nach 4 Stunden die Kristalle ab und wäscht mit Ethanol und Ether nach. Ausbeute: 20 g.

Diese werden in 166 ml THF mit 22,6 g Soda gerührt. Dann tropft man langsam 19 g Ölsäurechlorid in 20 ml THF bei 25 °C zu. Nach einer weiteren Stunde wird abgesaugt, das Filtrat im Vakuum zum Sirup eingedampft und das Rohprodukt

säulenchromatographisch an Kieselgel mit dem Elutionsmittel $CH_2Cl_2/CH_3OH$ = 13/1 gereinigt. Rf.-Wert der Titelverbindung = 0,53 in $CH_2Cl_2/CH_3OH$ = 13/2.

Beispiel 13:
N-Glucopyranosyl-N-tetradecyl-Ölsäureamid
Herstellung analog Beispiel 12.
Säulenchromatographie mit dem Elutionsmittel $CH_2Cl_2/CH_3OH$ = 13/1
$[\alpha]_D^{20}$ = + 9,6 (c=1,0 DMF)
Elementaranalyse:
ber. C = 70,3%, H = 11,3%, N = 2,16%
gef. C = 69,4%, H = 11,6%, N = 2,1%

Beispiel 14:
N-Glucopyranosyl-N-hexadecyl-Ölsäureamid
Herstellung und Reinigung analog Beispiel 12
Rf.-Wert 0,25 Laufmittel $CH_2Cl_2/CH_3OH$ = 13/1

Beispiel 15:
N-Glucopyranosyl-N-octadecyl-Ölsäureamid
90 g D-Glucose und 135 g Octadecylamin werden in 1000 ml Propanol – 2 und 500 ml Wasser auf 50 °C unter Rühren erhitzt bis eine klare Lösung entstanden ist. Dann beläßt man über Nacht bei Raumtemperatur. Nun wird das Produkt abgesaugt, mit Alkohol und Ether gewaschen, getrocknet und zuletzt aus Ethanol/THF umkristallisiert. 10 g dieses N-Octadecyl-β-D-glucopyranosylamins werden in 80 ml THF aufgeschlämmt und nach Zusatz von 10 g Soda tropfenweise mit 7 g Ölsäurechlorid in 10 ml THF versetzt. Nach quantitativer Umsetzung (DC in $CH_2Cl_2/CH_3OH$ = 13/1) wird wie in Beispiel 12 beschrieben aufgearbeitet. Säulenreinigung und Elutionsmittel $CH_2Cl_2/CH_3OH$ = 13/1.
RF.-Wert = 0,35 Laufmittelsystem $CH_2Cl_2/CH_3OH$ = 9/1.

Beispiel 16:
N-Glucopyranosyl-N-Octadecyl-Stearinsäureamid
Herstellung analog Beispiel 6 aus N-Octadecylglucopyranosylamin und Stearinsäurechlorid.
Elementaranalyse:
ber.: C = 72,5%, H = 11,7%, N = 2,0%
gef.: C = 71,7%, H = 12,2%, N = 2,0%

Beispiel 17:
N-Glucosyl-N-octadecyl-dodecansäureamid
Herstellung analog Beispiel 16 aus N-Octadecyl-β-D-glucopyranosylamin und Dodecansäurechlorid.
$[\alpha]_D^{20}$ = + 8° (C=1,0 Dioxan)

Beispiel 18:
N-Glucosyl-N-octadecyl-tetradecansäureamid
Herstellung analog Beispiel 16 aus N-Octadecyl-β-D-glucopyranosylamin und Tetradecansäurechlorid
$[\alpha]_D^{20}$ = + 9,5° (C=1,0 DMF)
Elementaranalyse:
ber.: C = 71,8%, H = 11,7%, N = 2,1%
gef.: C = 71,3%, H = 11,9%, N = 1,9%

Beispiel 19:
N-(2-Acetamido-2-desoxy-D-glucopyranosyl)-N-dodecyl-ölsäureamid
15 g N-Acetyl-D-glucosamin und 18,8 g Dodecylamin werden für 3 Std. in 50 ml Ethanol auf 80 °C unter Rühren erhitzt. Dann wird heiß vom Ungelösten abfiltriert, das Filtrat abgekühlt, das ausgefallene Produkt abgesaugt und mit Ethanol und Ether gewaschen 2,2 g des so erhaltenen 2-Acetamido-2-desoxy-N-octadecyl-glucopyranosylamins
versetzt man tropfenweise mit 1,45 g Ölsäurechlorid in 5 ml THF.
Aufarbeitung wie in Beispiel 6 beschrieben.
Säulenchromatographie mit dem Elutionsmittel $CH_2Cl_2/CH_3OH$ = 20/1.
$[\alpha]_D^{20}$ = + 9,2° (c= 0,56 $CH_3OH$)
Elementaranalyse:
ber.: C = 72,8%, H = 11,7%, N = 3,8%
gef.: C = 72,9%, H = 12,5%, N = 3,3%

Beispiel 20:
N-Octadecyl-L-rhamnopyranosylamin
9 g L-Rhamnose und 13,5 g Stearylamin werden in 100 ml Propanol – 2 und 50 ml Wasser bei 50 °C solange gerührt, bis eine klare Lösung entstanden ist. Nach 50 Stunden bei Raumtemperatur werden die Kristalle abgesaugt, mit Ethanol und Ether gewaschen und im Vakuum getrocknet. Ausbeute: 17,4 g.

Beispiel 21:
N-Octadecyl-N-rhamnopyranosyl-ölsäureamid
7 g der Verbindung aus Beispiel 20 werden wie in Beispiel 6 beschrieben mit Ölsäurechlorid acyliert. Säulentrennung in $CH_2Cl_2/CH_3OH$ = 13/1.
Elementaranalyse:
ber.: C = 74,4%, H = 11,9%, N = 2,04%
gef.: C = 74,3%, H = 12,0%, N = 2,1%

Beispiel 22:
N-Octadecyl-L-fucopyranosylamin
3,26 g L-Fucose und 5,38 g Stearylamin werden in 20 ml Ethanol auf 70 °C unter Rühren erhitzt, bis eine klare Lösung entstanden ist. Man läßt abkühlen, saugt den Feststoff nach beendeter Kristallisation ab und wäscht ihn mit Ethanol und Ether. Ausbeute nach Trocknen in Vakuum: 4,4 g.

Beispiel 23:
N-Fucopyranosyl-N-octadecyl-ölsäureamid
2,9 g der Verbindung aus Beispiel 22 werden wie in Beispiel 6 beschrieben mit Ölsäurechlorid acyliert. Säulenchromatographie mit dem Elutionsmittel $CH_2Cl_2/CH_3OH$ = 15/1.
Ausbeute an Reinprodukt: 1,9 g
RF-Wert = 0,44 Laufmittelsystem wie bei Säulenchromatographie.

Beispiel 24:
N-(β-D-Arabinopyranosyl)-N-octadecyl-ölsäureamid
7 g N-Octadecyl-β,D-arabinopyranosylamin werden wie in Beispiel 6 beschrieben mit Ölsäurechlorid acyliert. Säulenchromatographie mit Elutionsmittel $CH_2Cl_2/CH_3OH$ = 20/1.
Ausbeute an Reinprodukt: 2,3 g
RF-Wert = 0,57 Laufmittel $CH_2Cl_2/CH_3OH$ = 15/1.
$[\alpha]_D^{20}$ = + 20° (c = 1,03 $CH_2CC_2$)

Beispiel 25:
N-(β,D-Maltosyl)-N-octadecyl-ölsäureamid

3,04 g N-Octadecyl-β-D-maltosylamin werden wie in Beispiel 6 beschrieben mit Ölsäurechlorid acyliert.
Säulenchromatographie in $CH_2Cl_2/CH_3OH$ = 10/1.
RF.-Wert: 0,24 Laufmittel $CH_2Cl_2/CH_3OH$ = 8,1
$[\alpha]_D^{20}$ = + 22 °C (c = 0,5 $CH_3OH$)

Beispiel 26:
N-(4-Azido-4-desoxy-D-glucopyranosyl)-N-octadecyl-dodecansäureamid

3,09 g 4-Azido-4-desoxy-D-glucose werden in 30 ml Isopropanol und 15 ml Wasser gelöst und nach Zugabe von 4,05 g Octadecylamin auf 50 °C erwärmt.

Die entstandene Lösung wird über Nacht bei Raumtemperatur stehengelassen. Der entstandene Feststoff wird abfiltriert, mit wenig Ethanol und Ether nachgewaschen und getrocknet.

2,3 g dieses Produktes werden in 10 ml THF gelöst, mit 3 g Natriumcarbonat und 1,2 g Dodecansäurechlorid, gelöst in 15 ml THF, versetzt. Nach quantitativer Umsetzung wird wie bei Beispiel 12 beschrieben aufgearbeitet.
Rf.-Wert: 0,27 in $CH_2Cl_2/CH_3OH$ = 4 : 1 (V/V)

Beispiel 27:
N-(4-Acetamido-4-desoxy)-D-glucopyranosyl-N-octadecyl-ölsäureamid

3 g der Verbindung aus Beispiel 26 werden in 30 ml Dioxan/Methanol = 2/1 und 3 ml Acetanhydrid in Gegenwart von 1,0 g Palladiumkohle (5%) bei Normaldruck hydriert. Nach Beendigung der Reaktion (Laufmittelsystem $CH_2Cl_2/CH_3OH$ = 3/1) wird vom Katalysator abfiltriert und im Vakuum eingeengt.
Rf.-Wert: 0,18 ($CH_2Cl_2$/MeOH, 10 : 1 V/V)

Beispiel 28:
N-(6-Desoxy-6-fluoro-D-glucopyranosyl)-N-octadecyl-ölsäureamid

18,2 g 6-Desoxy-6-fluor-D-glucose und 13,5 g Octadecylamin und 7 g Ölsäurechlorid werden wie in Beispiel 15 beschrieben umgesetzt und aufgearbeitet.
Rf.-Wert: 0,30 in $CH_2Cl_2/CH_3OH$ = 9/1

Beispiel 29:
N-(methyl-D-glucopyranosyl uronato)-N-octadecyl-ölsäureamid

15 g D-Glucuronolacton werden in 150 ml abs. Methanol gelöst und mit 3 ml 1 N Natriummethanolat-Lösung eine halbe Stunde bei Raumtemperatur stehengelassen. Anschließend wird mit saurem Ionenaustauscher neutralisiert und eingedampft. Der entstandene Glucuronsäuremethylester wird wie bei Beispiel 15 beschrieben zur Titelverbindung umgesetzt und aufgearbeitet.
Rf.-Wert: 0,32 ($CH_2Cl_2/CH_3OH$ = 9 : 1, V/V)

Beispiel 30:
N-(Glucuronopyranosyl)-N-octadecyl-ölsäureamid

2 g der in Beispiel 29 beschriebenen Verbindung werden in 10 ml Dioxan gelöst und nach Zugabe von 5 ml 1 N Natronlauge 2 h am Rückfluß erhitzt. Nach dem Abkühlen wird mit verd. Salzsäure neutralisiert, im Vakuum eingeengt und der Rückstand mit 20 ml Methanol/Dioxan = 1/1 gerührt. Anschließend wird filtriert und das Filtrat zum Sirup eingeengt.
Rf.-Wert: 0,13 ($CH_2Cl_2/CH_3OH$ = 7 : 1, V/V)

Beispiel 31:
N-(4-Amino-4-desoxy-D-glucopyranosyl)-N-octadecyl-laurinsäureamid

3 g der Verbindung aus Beispiel 26 werden in 30 ml Dioxan/Methanol 2/1 in Gegenwart von 1,0 g Palladiumkohle (5%) hydriert. Nach Beendigung der Reaktion wird vom Katalysator abfiltriert und i. Vac. eingeengt.
Rf.-Wert: 0,39 $CH_2Cl_2$/MeOH 5 : 1

Beispiel 32:
N-(4-laurinamido-4-desoxy-D-glucopyranosyl)-N-octadecyl-laurinsäureamid

4,00 g in Beispiel 31 beschriebenen Verbindung werden in 30 ml THF gelöst, mit 2,0 g Natriumcarbonat versetzt und mit 1,42 g Dodecansäurechlorid in 10 ml THF umgesetzt. Nach 30 Min. wird mit Dichlormethan verdünnt, filtriert und das Filtrat i. Vac. eingeengt. Der Sirup wird säulenchromatographisch (Laufmittel Dichlormethan/Methanol = 15 : 1) gereinigt.
Rf.-Wert: 0,36 $CH_2Cl_2$/MeOH 10 : 1

Beispiel 33:
N-Glucopyranosyl-N-octadecyl-palmitinsäureamid

Herstellung analog Beispiel 16 aus N-Octadecyl-glucopyranosylamin und Palmitinsäurechlorid.
Rf.-Wert: 0,36 $CH_2Cl_2$/MeOH 9 : 1

Beispiel 34:
N-Octadecyl-N-glucopyranosyl-laurinsäureamid

Herstellung analog Beispiel 16 aus N-Octadecyl-glucopyranosylamin und Laurinsäurechlorid.
Rf.-Wert: 0,35 $CH_2Cl_2/CH_3OH$ 9 : 1

Beispiel 35:
N-Octadecyl-N-rhammopyranosyl-stearinsäureamid

Herstellung analog Beispiel 21 aus N-Octadecyl-rhammopyranosylamin und Stearinsäurechlorid.
Rf.-Wert: 0,39 $CH_2Cl_2/CH_3OH$ 9 : 1

Beispiel 36:
N-Octadecyl-(2-Amino-2-desoxy-D-glucopyranosyl)amin Hydrochlorid

6,45 g D-Glucosaminhydrochlorid werden bei 60° in 30 ml iso-Propanol und 10 ml Wasser gelöst

und mit 12,1 g Stearylamin versetzt. Die entstandene klare Lösung wird noch 10 min weiter gerührt und dann auf Raumtemp. abgekühlt. Das auskristallisierte Produkt wird abgesaugt und zunächst mit Ethanol/Wasser (5 : 2, v/v), dann mit Ethanol und schließlich mit Ether nachgewaschen. Der Rückstand wird am Hochvakuum getrocknet.

Beispiel 37:
N-Octadecyl-N-(2-dodecylamido-2-desoxy-D-glucopyranosyl)-dodecansäureamid

4,6 g der in Beispiel 36 beschriebenen Verbindung werden in 120 ml Tetrahydrofuran aufgeschlämmt und mit 22,6 g Natriumcarbonat versetzt. Zu der gerührten Suspension werden 4,2 g Dodecansäurechlorid in 20 ml Tetrahydrofuran zugetropft. Der Ansatz wird i. vac. eingedampft, mit 50 ml Pyridin und 25 ml Acetanhydrid acetyliert, auf Eiswasser gegossen, in Dichlormethan aufgenommen, die organische Phase wird nacheinander mit verdünnter Salzsäure, gesättigter Natriumhydrogencarbonatlösung und dann mit Wasser gewaschen, über Natriumsulfat getrocknet und i. vac. zum Sirup eingedampft. Der erhaltene Sirup wird säulenchromatographisch gereinigt (Laufmittel Toluol/Essigester = 10 : 1, v/v). Der erhaltene Feststoff (Schmp. 86°) wird in abs. Methanol gelöst, mit 20 mg Natriummethoxid versetzt und 20 min unter Rückfluß erhitzt. Nach Beendigung der Reaktion wird mit saurem Ionenaustauscher neutralisiert und i. vac. eingedampft.
Schmelzpunkt: 78 °C, Rf.-Wert: 0,64 in $CH_2Cl_2$/MeOH = 10/1 (v/v)

Beispiel 38:
N-Propyl-(2-amino-2-desoxy-D-glucopyranosyl)-amin-Hydrochlorid

21,5 g Glucosaminhydrochlorid werden in 17,7 g n-Propylamin aufgeschlämmt und auf 70 °C erwärmt, bis eine klare Lösung entsteht. Beim Abkühlen auf Rt. fiel das Produkt aus.

Beispiel 39:
N-Propyl-N-(2-ölsäureamido-2-desoxy-D-glucopyranosyl)ölsäureamid

5,1 g der in Beispiel 38 beschriebenen Verbindung werden in 100 ml Tetrahydrofuran aufgeschlämmt, mit 12,7 g Natriumcarbonat versetzt. Anschließend werden 12 g Ölsäurechlorid in 20 ml Tetrahydrofuran zugetropft. Nach Beendigung der Reaktion wird der Ansatz mit 50 ml Dichlormethan verdünnt, vom Natriumsalz abfiltriert, mit Wasser gewaschen, getrocknet über Natriumsulfat und i. vac. eingedampft. Der erhaltene Sirup wird säulenchromatographisch gereinigt (Laufmittel Dichlormethan/Methanol 15/1, v/v).
Rf.-Wert: 0,37 in $CH_2Cl_2$/MeOH 10 : 1
$\alpha_D$ = 17,9° (c = 1,02 in Dichlormethan)

Beispiel 40:
N-Glucopyranosyl-N-tetradecyl-stearinsäureamid

Herstellung analog Beispiel 11 aus N-Tetradecyl-glucopyranosylamin und Stearinsäurechlorid.
Rf.-Wert: 0,25 in Toluol/Aceton 1 : 1

Beispiel 41:
N-Dodecyl-N-(2-amino-2-desoxy-glucopyranosyl)-amin Hydrochlorid

46 g Dodecylamin werden bei 60° aufgeschmolzen und unter Rühren mit 31 g Glucosaminhydrochlorid versetzt. Nach Abkühlen auf Rt. fällt das Produkt aus. Der Feststoff wird dreimal mit Ether aufgerührt und abgesaugt und anschließend im Hochvakuum getrocknet.

Beispiel 42:
N-Dodecyl-N-(2-stearylamido-2-desoxy-D-glucopyranosyl)-stearinsäureamid

5 g der in Beispiel 41 beschriebenen Verbindung werden in 100 ml Tetrahydrofuran aufgeschlämmt, mit 8,5 g Natriumcarbonat und 8 g Stearinsäurechlorid in 20 ml Tetrahydrofuran versetzt. Nach Beendigung der Reaktion wird wie bei Beispiel 39 beschrieben aufgearbeitet. Der erhaltene Rohsirup wird aus Essigester kristallisiert.
Schmp. 67°;
Rf.-Wert 0,42 in $CH_2Cl_2$/MeOH 10/1

Beispiel 43:
N-Dodecyl-N-(2-laurinsäureamido-2-desoxy-D-glucopyranosyl)-laurinsäureamid

5 g der in Beispiel 41 beschriebenen Verbindung werden wie bei Beispiel 42 beschrieben mit Laurinsäurechlorid umgesetzt.
Schmp. 67°;
Rf.-Wert 0,42 in $CH_2Cl_2$/MeOH 10/1

Beispiel 44:
N-Octadecyl-N-(galactopyranosyl)-amin

60 g D-Galactose werden in 330 ml Isopropanol und 170 ml Wasser suspendiert und auf 50° erwärmt. Nach Zugabe von 90 g Stearylamin wird so lange gerührt, bis alles Amin in Lösung gegangen ist. Beim Erkalten kristallisiert das Glycosylamin aus. Der Feststoff wird abgesaugt, nacheinander mit Ethanol und mit Ether gewaschen und i. vac. getrocknet.

Beispiel 45:
N-Octadecyl-N-(D-galactopyranosyl)-laurinsäureamid

Herstellung aus 8,4 g der in Beispiel 44 beschriebenen Verbindung und 4,4 g Dodecansäurechlorid analog Beispiel 11.
Rf.-Wert: 0,22 in Toluol-n-Propanol 4/1 (v/v)
$\alpha_D$ = 11,4° (c = 0.93 in Dichlormethan)

Beispiel 46:
N-Tetradecyl-N-(D-galactopyranosyl)-ölsäureamid

Aus 30 g D-Galactose und 53 g Tetradecylamin wird wie bei Beispiel 44 beschrieben N-Tetradecyl-N-(D-galactopyranosyl)-amin hergestellt. Das Galactoxylamin wird nach dem in Beispiel 11 beschriebenen Verfahren mit Ölsäurechlorid umgesetzt.
Rf.-Wert: 0,26 in Toluol/n-Propanol 4/1 (v/v)
$\alpha$ = 11° (c = 1.0 in Dichlormethan)

Beispiel 47:
N-Octadecyl-N-mannopyranosyl-amin

20 g D-Mannose und 45 g Stearylamin werden wie in Beispiel 44 beschrieben zum Glycosylamin umgesetzt.

Beispiel 48:
N-Octadexyl-N-(D-mannopyranosyl)-laurinsäureamid

8,6 g der bei Beispiel 47 beschriebenen Verbindung werden mit 4,4 g Dodecansäurechlorid wie bei Beispiel 11 beschrieben umgesetzt.
Rf.-Wert: 0,25 in Toluol/n-Propanol 4/1 (v/v)
$\alpha_D$ = 11,3° (c = 1,13 in Dichlormethan)

Beispiel 49:
N-Octadecyl-N-(D-mannopyranosyl)-tetradecansäureamid

Herstellung aus der unter Beispiel 47 beschriebenen Verbindung und Tetradecansäurechlorid analog zu Beispiel 11.
Rf.-Wert: 0,26 in Toluol/n-Propanol 4/1 (v/v)
α = 9,9° (c = 1,0 in Dichlormethan)

Beispiel 50:
N-Tetradecyl-N-(D-mannopyranosyl)-ölsäureamid

20 g D-Mannose und 35 g Tetradecylamin werden wie bei Beispiel 44 beschrieben zum N-Tetradecyl-mannopyranosylamin umgesetzt. In einem zweiten Reaktionsschritt wird das Glycosylamin (7,5 g) mit 6,0 g Ölsäurechlorid wie im Beispiel 11 beschrieben zum Glycosylamid umgesetzt.
Rf.-Wert: 0,29 in Toluol/n-Propanol 4/1 (v/v)
α = 10,8° (c = 1 in Tetrahydrofuran)

Beispiel 51:
2-Dodecylamido-2-desoxy-D-glucopyranose

55 g Dodecansäurechlorid werden in 170 ml Tetrahydrofuran gelöst und unter starkem Rühren zu einer Lösung von 54 g D-Glucosaminhydrochlorid in 330 ml wäßriger Natriumcarbonatlösung (20%) getropft. Nach Beendigung der Säurechloridzugabe wird noch eine Stunde weitergerührt, dann wird der Ansatz mit 500 ml Wasser versetzt und der Feststoff abgesaugt und mit Wasser gewaschen. Der Rückstand wird aus iso-Propanol/Wasser 10/1 (v/v) umkristallisiert und im Hochvakuum getrocknet.

Beispiel 52:
N-Dodecyl-N-(2-dodecylamido-2-desoxy-D-glucopyranosyl)-amin

15 g der in Beispiel 51 beschriebenen Verbindung werden mit 45 g Dodecylamin und 75 ml Ethanol versetzt und unter Rühren auf 70° erwärmt. Nachdem sich eine klare Lösung gebildet hat, wird auf Raumtemp. abgekühlt und über Nacht kristallisiert. Der ausgefallene Feststoff wird abgesaugt, einmal mit Ethanol und dreimal mit Ether gewaschen und i. vac. getrocknet.

Beispiel 53:
N-Dodecyl-N-(2-dodecylamido-2-desoxy-D-glucopyranosyl)-stearinsäureamid

4 g der in Beispiel 52 beschriebenen Verbindung werden in 100 ml Tetrahydrofuran gelöst und mit 4,8 g Natriumcarbonat versetzt. Zu dieser Suspension werden unter Rühren 3,45 g Stearinsäurechlorid in 20 ml Tetrahydrofuran gelöst zugetropft. Es wird 30 min weitergerührt, dann wird mit 50 ml Dichlormethan verdünnt und vom Feststoff abgesaugt. Der Rückstand wird mit Dichlormethan gewaschen. Die organischen Lösungsmittelphasen werden vereinigt und i. vac. eingeengt. Der erhaltene Sirup wird chromatographisch gereinigt. (Laufmittel: Dichlormethan/Methanol 20/1 (v/v)).

Rf.-Wert: 0,55 in Dichlormethan/Methanol 10/1 (v/v)
α = 15,8° (c = 1,05 in Dichlormethan)

Beispiel 54:
N-Dodecyl-N-(2-acetamido-2-desoxy-D-glucopyranosyl)-tetradecansäureamid

26 g N-Acetylglucosamin werden in 100 ml Ethanol und 60 ml Wasser gelöst und auf 60° erwärmt. Es werden 37 g Dodecylamin hinzugegeben und bis zur Einstellung einer klaren Lösung gerührt. Nach dem Abkühlen auf Raumtemp. kristallisiert das Glycosylamin aus. Der Kristallbrei wird abgesaugt, mit Ethanol und dann mit Ether gewaschen und i. vac. getrocknet. 3 g des Feststoffes werden in 50 ml Tetrahydrofuran aufgeschlämmt, mit 3,3 g Natriumcarbonat versetzt und mit 1,9 g Tetradecansäurechlorid in 10 ml Tetrahydrofuran versetzt. Nach Beendigung der Reaktion wird mit 30 ml Dichlormethan verdünnt, filtriert und das Filtrat i. vac. eingedampft. Der erhaltene Sirup wird chromatographisch gereinigt (Laufmittel Dichlormethan/Methanol 20/1, (v/v)).
Rf.-Wert: 0,21 in Dichlormethan/Methanol 10/1 (v/v)

Beispiel 55:
N-Dodecyl-N-(2-acetamido-2-desoxy-D-glucopyranosyl)-stearinsäureamid

3 g N-(2-Acetamido-2-desoxy)-dodecylamin, dessen Darstellung in Beispiel 54 beschrieben ist, werden wie in Beispiel 54 beschrieben mit Stearinsäurechlorid umgesetzt.
Rf.-Wert: 0,23 in Dichlormethan/Methanol 10/1 (v/v)

Beispiel 56:
N-Octadecyl-N-(2-acetamido-2-desoxy-D-glucopyranosyl)-tetradecansäureamid

Herstellung analog Beispiel 19 aus N-Acetylglucosamin, Stearylamin und Tetradecansäurechlorid.
Rf.-Wert: 0,25 in Toluol/iso-Propanol 4/1 (v/v)
α = 16,9° (c = 1 in Tetrahydrofuran)

Beispiel 57:
N-Dodecyl-N-(D-mannopyranosyl)-stearinsäureamid

Herstellung aus D-Mannose, Dodecylamin und Stearinsäurechlorid analog Beispiel 11.
Rf.-Wert: 0,28 in Toluol/n-Propanol 4/1 (v/v)
α = 11,4° (c = 1 in Tetrahydrofuran)

Beispiel 58:
N-Dodecyl-N-(D-galactopyranosyl)-stearinsäureamid

Herstellung aus D-Galactose, Dodecylamin und Stearinsäurechlorid analog Beispiel 11.
Rf.-Wert: 0,28 in Toluol/n-Propanol 4/1 (v/v)
$\alpha$ = 4,4° (c = 1 in Dichlormethan)

Beispiel 59:
N-Dodecyl-N-(2-acetamido-2-desoxy-D-glucopyranosyl)-dodecansäureamid

Herstellung analog Beispiel 54 aus N-Acetylglucosamin, Dodecylamin und Dodecansäurechlorid.
Rf.-Wert: 0,21 in Dichlormethan/Methanol 10/1 (v/v)

Beispiel 60:
N-Dodecyl-N-(D-ribopyranosyl)-dodecansäureamid

10 g D-Ribose werden in 180 ml Isopropylalkohol/Wasser 2/1 (v/v) gelöst und auf 70 °C erwärmt. Es werden 18 g Dodecylamin zugegeben. Nachdem sich eine klare Lösung gebildet hat, wird die Temperatur noch 15 min beibehalten. Es wird auf Raumtemperatur abgekühlt und i. vac. eingedampft. Von dem erhaltenen Sirup werden 10 g in 100 ml Tetrahydrofuran und 10 ml Methanol gelöst. Nach Zugabe von 10 g Natriumcarbonat wird auf 0 °C gekühlt. Es werden 5,5 g Dodecansäurechlorid, gelöst in 20 ml Tetrahydrofuran, zugetropft. Nach Beendigung der Reaktion wird wie üblich aufgearbeitet und das Produkt durch Säulenchromatographie gereinigt.
$\alpha_D$ = 5,3° (c = 1,1 in Tetrahydrofuran)

**Patentansprüche**

1. Verwendung von Verbindungen der allgemeinen Formel I

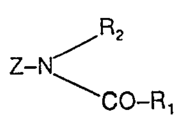

worin

Z seinen über das anomere Kohlenstoffatom gebundenen Glycosylrest

$R_1$ Wasserstoff oder einen gegebenenfalls substituierten geradkettigen oder verzweigten, gesättigten oder ein- oder mehrfach ungesättigten Kohlenwasserstoffrest mit bis zu 30 C-Atomen, wobei dieser Rest auch durch O, N oder S unterbrochen sein kann und

$R_2$ Wasserstoff oder einen geradkettigen oder verzweigten, gesättigten oder ein- oder mehrfach ungesättigten Alkyl- oder Aralkylrest mit bis zu 30 C-Atomen, der durch O unterbrochen oder durch Sauerstoff enthaltende Gruppen oder Halogen substituiert sein kann

bedeuten mit der Maßgabe, daß $COR_1$ keine Acylgruppen mit 1–5 C-Atomen darstellt, wenn $R_2$ Alkyl mit 10–20 C-Atomen bedeutet, zur Förderung und Beschleunigung des Wachstums von Tieren.

2. Verwendung von Verbindungen der Formel I gemäß Anspruch 1, worin

$R_2$ einen Alkyl oder Alkenylrest mit 1–20 C-Atomen darstellt, zur Förderung und Beschleunigung des Wachstums von Tieren.

3. Verwendung von N-Octadecyl-N-D-glucopyranosyl-laurinsäureamid, N-Octadecyl-N-D-glucopyranosyl-ölsäureamid oder N-Dodecyl-N-(2-acetamido-2-desoxy-D-Glucopyranosyl)-dodecansäureamid zur Förderung und Beschleunigung des Wachstums von Tieren.

4. Tiernahrung zur Förderung und Beschleunigung des Wachstums von Tieren enthaltend Verbindungen der Formel I nach Anspruch 1.

5. Verfahren zur Herstellung von wachstumsfördernder Tiernahrung, dadurch gekennzeichnet, daß man der Tiernahrung Verbindungen der allgemeinen Formel I in Anspruch 1 zusetzt.

6. Praemixe zur Herstellung von Tiernahrung zur Förderung und Beschleunigung des Wachstums bei Tieren enthaltend Verbindungen der allgemeinen Formel I gemäß Anspruch 1.

7. N-Dodecyl-N-(2-acetamido-2-desoxy-D-glucopyranosyl)-dodecansäureamid.

8. N-Dodecyl-N-(D-ribopyranosyl)-dodecansäureamid.

**Claims**

1. Use of compounds of the general formula I

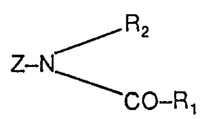

in which

Z denotes its glycosyl radical bonded via the anomeric carbon atom,

$R_1$ denotes hydrogen or an optionally substituted, straight-chain or branched, saturated or singly or multiply unsaturated hydrocarbon radical having up to 30 C atoms, it being possible for this radical also to be interrupted by O, N or S, and

$R_2$ denotes hydrogen or a straight-chain or branched, saturated or singly or multiply unsaturated alkyl or aralkyl radical having up to 30 C atoms, which can be interrupted by O or substituted by groups containing oxygen or by halogen, with the proviso that $COR_1$ does not represent acyl groups having 1–5 C atoms when $R_2$ denotes alkyl having 10–20 C atoms,

for promoting and accelerating the growth of livestock.

2. Use of compounds of the formula I according to Claim 1, in which

$R_2$ represents an alkyl or alkenyl radical having 1–20 C atoms,

for promoting and accelerating the growth of livestock.

3. Use of N-octadecyl-N-D-glucopyranosyllauramide, N-octadecyl-N-D-glucopyranosyloleamide or N-dodecyl-N-(2-acetamido-2-deoxy-D-glucopyranosyl)dodecanoamide for promoting and accelerating the growth of livestock.

4. Livestock food for promoting and accelerating the growth of livestock containing compounds of the formula I according to Claim 1.

5. Process for the preparation of growth-promoting livestock food, characterized in that compounds of the general formula I in Claim 1 are added to the livestock food.

6. Premixes for the preparation of livestock food for promoting and accelerating growth in livestock, containing compounds of the general formula I in Claim 1.

7. N-Dodecyl-N-(2-acetamido-2-deoxy-D-glucopyranosyl)-dodecanoamide.

8. N-Dodecyl-N-(D-ribopyranosyl)dodecanoamide.

**Revendications**

1. Utilisation de composés de formule générale I

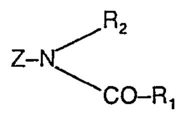

dans laquelle

Z est un reste glycosyle lié par l'intermédiaire d'un atome anomère de carbone

$R_1$ est l'hydrogène ou un reste à chaîne droite ou ramifiée, saturé ou à une ou plusieurs non-saturations, éventuellement substitué, ayant jusqu'à 30 atomes de carbone, ce reste pouvant aussi être interrompu par de l'oxygène, de l'azote ou du soufre et

$R_2$ est l'hydrogène ou un reste alkyle à chaîne droite ou ramifiée, saturé ou à une ou plusieurs non-saturations ou un reste aralkyle, avec jusqu'à 30 atomes de carbone, qui peut être interrompu par de l'oxygène ou substitué par des groupes contenant de l'oxygène ou par un halogène, sous réserve que $COR_1$ ne représente pas de groupes acyle ayant 1 à 5 atomes de carbone lorsque $R_2$ désigne un groupe alkyle de 10 à 20 atomes de carbone, pour l'activation et l'accélération de la croissance d'animaux.

2. Utilisation de composés de formule I suivant la revendication 1, dans laquelle

$R_2$ désigne un reste alkyle ou alcényle de 1 à 20 atomes de carbone, pour l'activation et l'accélération de la croissance d'animaux.

3. Utilisation du N-octadécyl-N-D-glucopyrannosylamide d'acide laurique, du N-octadécyl-N-D-glucopyrannosylamide d'acide oléique ou du N-dodécyl-N-(2-acétamido-2-désoxy-D-gluco-pyrannosylamide d'acide dodécanoïque pour activer et accélérer la croissance d'animaux.

4. Aliment pour animaux, destiné à activer et à accélérer la croissance d'animaux, contenant des composés de formule I suivant la revendication 1.

5. Procédé de préparation d'un aliment activateur de croissance pour animaux, caractérisé en ce qu'on ajoute à l'aliment pour animaux des composés de formule générale I suivant la revendication 1.

6. Mélange préalable pour la préparation d'un aliment pour animaux, destiné à activer et à accélérer la croissance d'animaux, contenant des composés de formule générale I suivant la revendication 1.

7. Le N-dodécyl-N-(2-acétamido-2-désoxy-D-glucopyrannosyl)amide d'acide dodécanoïque.

8. Le N-dodécyl-N-(D-ribopyrannosyl)amide d'acide dodécanoïque.